# EUROPEAN PATENT SPECIFICATION

(11) **EP 3 235 541 B1**
(45) Date of publication and mention of the grant of the patent: **13.02.2019**
(21) Application number: 17162572.6
(22) Date of filing: 23.03.2017
(51) Int. Cl.: A61N 1/36

(54) **PROCESS FOR ESTABLISHING AN ELECTROSTIMULATION PROTOCOL, AND RESPECTIVE ELECTROSTIMULATION PORTABLE EQUIPMENT USING SAID PROTOCOL**
VERFAHREN ZUR ERSTELLUNG EINES ELEKTROSTIMULATIONSPROTOKOLLS UND ZUGEHÖRIGE TRAGBARE ELEKTROSTIMULATIONSAUSRÜSTUNG MIT VERWENDUNG DES BESAGTEN PROTOKOLLS
PROCÉDÉ D'ÉTABLISSEMENT D'UN PROTOCOLE D'ÉLECTROSTIMULATION ET ÉQUIPEMENT D'ÉLECTROSTIMULATION PORTABLE RESPECTIF UTILISANT LEDIT PROTOCOLE

(30) Priority: 31.03.2016 BR 102016007239
(43) Date of publication of application: 25.10.2017
(73) Proprietor: Medecell S.A., Montevideo (UY)
(72) Inventor: Marques de Oliveira, Mauricio, São Paulo (BR); Bighetti, Moacyr Ramos, São Paulo (BR)
(74) Representative: Michalski Hüttermann & Partner Patentanwälte mbB

(56) References cited:
- WO-A1-2007/018793
- US-A1- 2008 208 287
- US-A1- 2011 009 919

## Description

### TECHNICAL FIELD

As known in the state of the art, Transcutaneous Electrical Nerve Stimulation (TENS) is a well-known and already consecrated modality of non-medicament and non-invasive treatment for pain control in several etiologies. Such treatment consists in the placement of electrodes in determined regions of the human body, and in the application of electric pulses with the purpose of stimulating the nerves fibers (or nerves); this electrical stimulation produces an analgesic effect generating a reduction or even total elimination of the pain.

This treatment mode has already been used in several clinical scenarios for the treatment of several acute and chronic pain conditions, and have been well-accepted among health professionals.

TENS is especially indicated in cases of painful disorders of the locomotor and nervous system, such as: arthritis, muscle inflammation, fibromyalgia, neuralgia, etc. Furthermore, chronic and recurrent pain, including visceral pain, such as those caused by dysmenorrhea and oncological origin, has also been successfully treated by electrostimulation. It is an alternative or an adjuvant to the medicated analgesic treatment, also reducing the need for anti-inflammatories.

### DESCRIPTION OF THE PRIOR ART

Among the countless electrostimulation equipment known in the prior art, let us cite the ones disclosed in the following documents: US 4.014.347, US 4.537.195, US 4.693.254, US 5.067.495, US 5.273.033, US 5.620.483, US 5.776.170, US 6.493.588, US 7.922.676 and US 8.700.177 Document US-A-2011/009919 discloses the most relevant prior art.

The usual electrostimulation equipment can be divided into two large groups: the bench ones, powered by the electric network, and the portable ones, powered by batteries. For the use of bench equipment, it is necessary to move the user to the places where the device is available, said device being usually operated by specialized people (physician or physiotherapists). On the contrary, the portable equipment is self-applicable and, after a professional has been indicated, the user can use it himself/herself in his/her home or work environment.

The portable equipment available in the market involves a high current consumption, causing the need for frequent exchange or recharge of the power source batteries. This is a drawback of common equipment.

It is also known that to be effective in the treatment of pain, electrical stimuli (or pulses) must meet a series of requirements in terms of their intensity (or amplitude) [in volts (V)], their frequency [in hertz (Hz)], their width (or duration) [in microseconds (µs)] and its waveform. Pulses can be temporally organized as continuous or intermittent.

In the scientific literature, there are several reports showing that analgesia induced by electrical pulses occurs within a very elastic range of the previously-mentioned parameters.

In fact, the numerous electrostimulation devices known in the art usually apply electrical pulses whose parameters are found in the following ranges:
- current intensity: 1-50 mA (with a 500Ω charge);
- frequency: 1-250 Hz;
- pulse-width (or duration): 10-1000 µs;
- Waveform: single-phase, symmetrical biphasic, or asymmetrical biphasic;
- stimulation mode: continuous or intermittent.

Although there is extensive literature attesting to the efficacy of TENS, the mechanism of action is not fully understood, and the Theory of the Pain Portal and the Central Release of Endorphins are the most accepted mechanisms of action by the scientific community.

A well-known physiological phenomenon is the one of the nerve fiber accommodation at the electric stimulus. It is the refractoriness of the nerve cell membrane when the stimulus is applied in the same phase and with fixed parameters of intensity, frequency, and pulse width. In this case, the stimulation ceases to be early effective, and the analgesic effect can be compromised.

To avoid the nerve fiber accommodation, several strategies have been developed, among them:
- inversion of the polarity of the electrical pulses;
- waveform change;
- variation of the frequency or intensity (amplitude) of the electrical pulses.

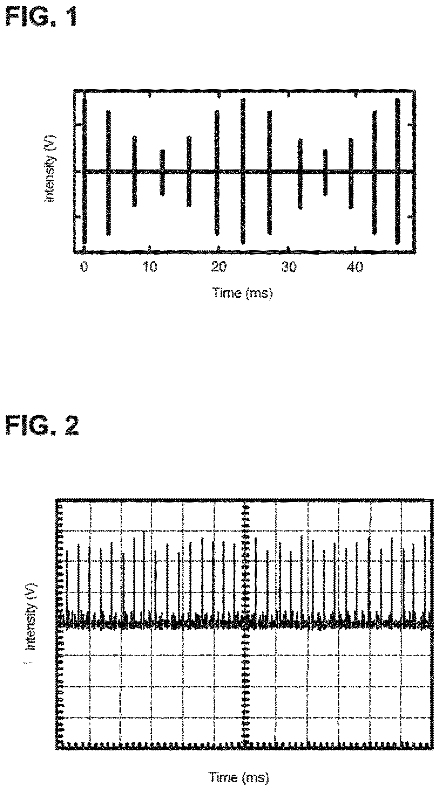

When the variation of the intensity (amplitude) of the electrical pulses is used as a strategy to prevent the accommodation of the nerve fibers, this variation is always done in a regular way in time (according to the graphical representation of attached Figure 1)

However, even when adopting this measure, there is still some degree of nerve fiber accommodation, precisely because there is a regular temporal repetition of said intensity variations. As a function of the plasticity and adaptive capacity of the cell membranes of the nerve fibers, regular intervals of variation allow said membranes to adapt, as well as cause the nerve fibers to be accommodated, thereby reducing the analgesic effect.

This is another inconvenient of the electrostimulation equipment currently available in the market: the difficulty in avoiding nerve fiber accommodation when applying the electrical pulses.

Therefore, it would be desirable to develop some kind of protocol for the application of electrical stimuli that would be able to mitigate the accommodation of the nerve fibers in a more efficient way, and, therefore, to guarantee the prolonged analgesic effect.

In addition, it would also be desirable to obtain portable equipment for electrostimulation application, which consumes less current when compared to the application of electrical pulses. This low consumption would allow the use of batteries of smaller size and capacity, reducing the production costs, increasing its portability and avoiding the need for battery replacement for an acceptable period of use for various therapeutic sessions. Hence, this equipment might be disposable.

### PURPOSES OF THE INVENTION

To achieve these purposes, an innovative electrostimulation protocol was developed, in which the intensity variation of the applied electrical pulses was randomly performed, respecting the limits stimulation efficacy. This new protocol has proven to be effective in reducing the accommodation of nerve fiber cell membrane, increasing electrostimulation effectiveness and, thus, the analgesic effect.

Concurrently, this random variation of electrical pulse intensity has further allowed to substantially reduce current consumption in the operations of the electrostimulation equipment. Thus, thanks to the innovative electrostimulation protocol, it was possible to use disposable coin-shaped lithium-ion batteries, model CR20XX, which have a sufficient size and charge to allow the development of a disposable and low-cost equipment.

Only this family of batteries combines the characteristics of reduced size, sufficient voltage and charge, low environmental impact and low cost, which makes it possible to dispose of the equipment.

Concurrently, since the use of such disposable batteries is now possible, the dimensions of the equipment have been substantially reduced, increasing its portability and ergonomics.

Therefore, the creation of this novel electrostimulation protocol has made possible the development of a disposable equipment of low cost, reduced sizes and great portability for the TENS application outside the hospital environment, capable of serving a greater number of patients who cannot move to an outpatient unit Therefore, the domestic use or the use in the work environment itself at any time has been made possible, avoiding unnecessary displacements, as well as the associated costs.

The generation of this aleatory (or random) variation of the pulse intensity is performed using a micro-controller and a specially developed software program, or using analog electronics, from suitable sizing of discrete components.

In this innovative protocol, other strategies are simultaneously used in conjunction with random variation of pulse intensity, not only to further prevent nerve fiber accommodation, but also to make current consumption even smaller, allowing the now-innovated equipment to be, as already mentioned, portable and disposable, with low cost, reduced sizes, and long operational time.

The following strategies are used simultaneously in conjunction with the aleatory (or random) variation of pulse intensity:
- the use of monopolar pulse bursts, which allow saving battery charge;
- polarity inversion of the pulse bursts, avoiding muscular fiber accommodation effect;
- inclusion of intermittent modes of stimulation for the maintenance of analgesia, once the desired effect has been achieved, saving the battery charge;
- selection of different bands of electrostimulation, all of them including the range of random variation.

### DESCRIPTION OF THE DRAWINGS

To complement the present description, to better understand the characteristics of the subject matter of the patent, a set of drawings accompanies this specification, in which, in an exemplified and nonlimiting manner, the following has been represented:
- Figure 1 is a graphical representation showing the usual strategy for avoiding the nerve fiber accommodation used in the known electrostimulation protocols, that is, the use of intensity (amplitude) variation of the electrical pulses, variation that, to this day, is done regularly over time;
- Figure 2 is another graphical representation, now illustrating the novel strategy to avoid nerve fiber accommodation, provided by this innovative electrostimulation protocol, that is, the use of a random variation of electrical pulse intensity (amplitude), during the application of pulse bursts;
- Figure 3 shows, also by means of a graphical representation, one of the embodiment of this innovative electrostimulation protocol, where pulse bursts with a determined duration and with sequentially inverted polarity are continuously applied;
- Figure 4 illustrates, similarly by means of a graphical representation, other embodiments of this innovative electrostimulation protocol, that is, the intermittent mode, according to which pulse bursts are applied with a determined duration, and with sequentially inverted polarity, although providing a time interval between said pulse bursts, with a determined duration.
- Figure 5 is a perspective view of the electrostimulation equipment, in which this innovative stimulation protocol is applied, wherein said equipment may exhibit any external configuration, among them, the shape of a band, such as illustrated, as an example, in said figure;
- Figure 6 is a block diagram of this innovative portable elecrostimulation equipment where said electrostimulation protocol is used, where said block diagram further includes the electrical scheme of the equipment;
- finally, Figure 7 is a flowchart of the software, specifically developed for this innovative electrostimulation protocol.

### DETAILED DESCRIPTION OF THE INVENTION

The present application for patent of disclosure relates to a "PROCESS FOR ESTABLISHING AN ELECTROSTIMULATION PROTOCOL, AND RESPECTIVE ELECTROSTIMULATION PORTABLE EQUIPMENT USING SAID PROTOCOL", said electrostimulation process and equipment being used to control pain in different etiologies, more in particular, painful disorders of the locomotor and nervous system (among them arthritis, muscle inflammation, fibromyalgia, neuralgia, etc.), and in cases of chronic and recurrent pain (among them the ones caused by dysmenorrhea and the ones of oncological origin).

Initially, with references to the "PROCESS FOR ESTABLISHING AN ELECTROSTIMULATION PROTOCOL", said process provides the development of an electrostimulation protocol in which intensity variation of the electrical pulses is performed in a random manner, respecting the limits of stimulation efficacy, to reduce the physiological phenomenon of nervous fiber accommodation upon receiving stimuli.

More specifically, according to the present process, the electrical pulses exhibit the shape of a square wave, are monopolar and have a width (or duration) between 60 µs and 100 µs, preferably 80 µs, frequency between 40 Hz and 70 Hz, preferably 55 Hz, and, for each intensity level chosen by the user, there is a random variation occurring from the selected limit to a lower one, in the range of 10 V, preferably 5 V (with a 500Ω charge), as shown in attached Figure 2.

Furthermore, the present process discloses two embodiments for this innovative electrostimulation protocol, namely, continuous mode and intermittent mode.

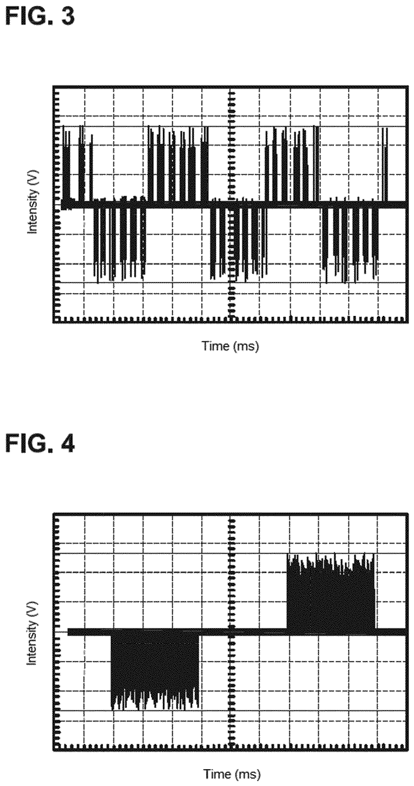

In the continuous mode, shown in the graphical representation of Figure 3, burst trains lasting 500 milliseconds to 2 seconds, preferably 1 second, and with sequentially inverted polarity are continuously applied.

In the intermittent mode, shown in the graphical representation of Figure 4, pulse bursts lasting from 2 to 4 seconds, preferably 3 seconds, and with sequentially inverted polarity are applied, however there is a time interval from 2 to 4 seconds, preferably 3 seconds.

In optional embodiments, this innovative protocol discloses the simultaneous use of other strategies used together with the random intensity pulse variation, as a complementary way to further reduce nerve fiber accommodation, as well as to make current consumption even smaller.

Among the strategies simultaneously employed with the aleatory (or random) variation of the pulse intensity, let us cite the following ones:
- the use of monopolar pulse bursts, which allow saving battery charge;
- polarity inversion of the pulse bursts, avoiding muscular fiber accommodation effect;
- inclusion of intermittent modes of stimulation for the maintenance of analgesia, once the desired effect has been achieved;
- selection of different bands of electrostimulation, all of them including the range of random variation.

By means of this new protocol, in which the intensity of the electric pulses has begun to vary in a random way, it was possible to sensibly reduce nerve fiber accommodation, increasing the effectiveness of the electrostimulation and, thus, of the analgesic effect.

Concurrently, this random variation of electric pulse intensity further allowed a substantial reduction in current consumption in the operations of the electrostimulation equipment, allowing the use of standard coin-shaped lithium ion batteries, model CR20XX, which are smaller, cheaper and of lower load capacity, but sufficient to meet the current lower current consumption due to random intensity variations, incorporated by this innovative electric stimulation protocol. Hence, battery replacement becomes unnecessary.

Thus, since it is now possible to use such common ion-lithium batteries, it has been possible to substantially reduce the dimensions of the equipment, which are greater because they are determined by higher capacity batteries until then necessary to provide electrostimulation operations of the apparatus.

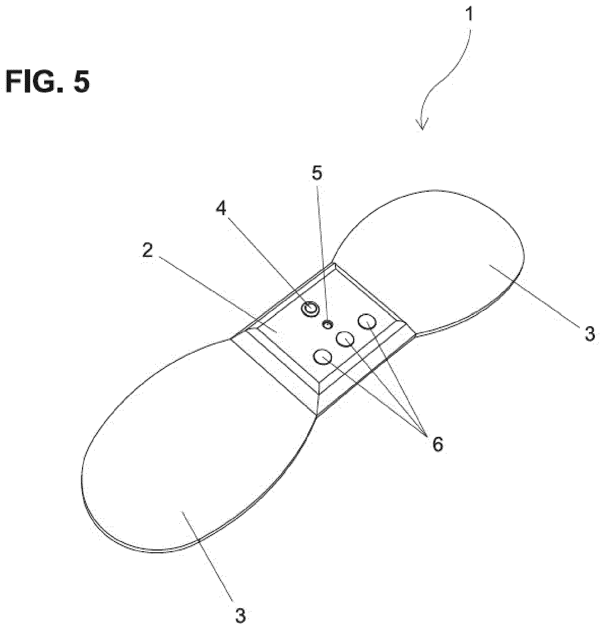

With references to the novel "ELECTROSTIMULATION PORTABLE EQUIPMENT USING SAID PROTOCOL", said equipment can exhibit any type of external configuration, among them, the one illustrated, as an example, in Figure 5, according to which the equipment usually consists of a bandage (1) comprising a central electronic module (2) and two side flaps (3), which contain, at the lower part, respective electrodes (not shown), suitably covered by the respective gel layers, that are, in turn, protected by the respective removable protective sheets (also not shown). The central electronic module (2) houses the internal components and the electrical circuit of the equipment, as well as its power supply battery, and an external power button (4), a led (5) indicating the functioning of the apparatus and one or more control buttons (6).

Thanks to the previously described innovative electrostimulation protocol, the power supply battery of this innovative equipment could now be a disposable coin-shaped lithium-ion battery, model CR20XX.

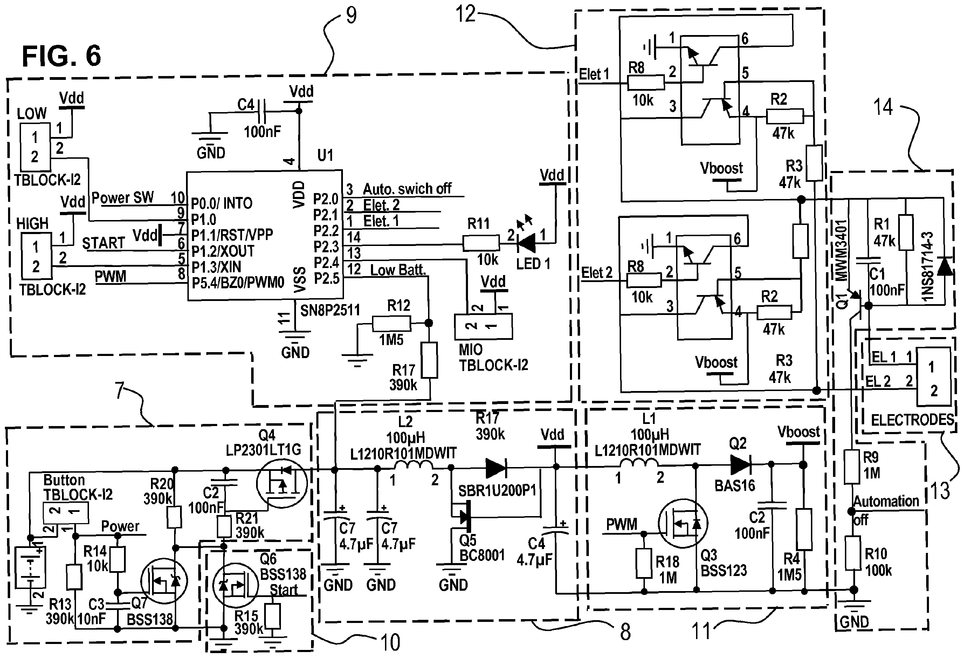

As shown in Figure 6, this innovative equipment, shows the following modules of internal components: Power source module (7), step-up regulator module (8), micro controller module (9), power supply seal module (10), boost source module (11), H-bridge module (12), electrode output module (13), and module for switching off when there is no charge (14).

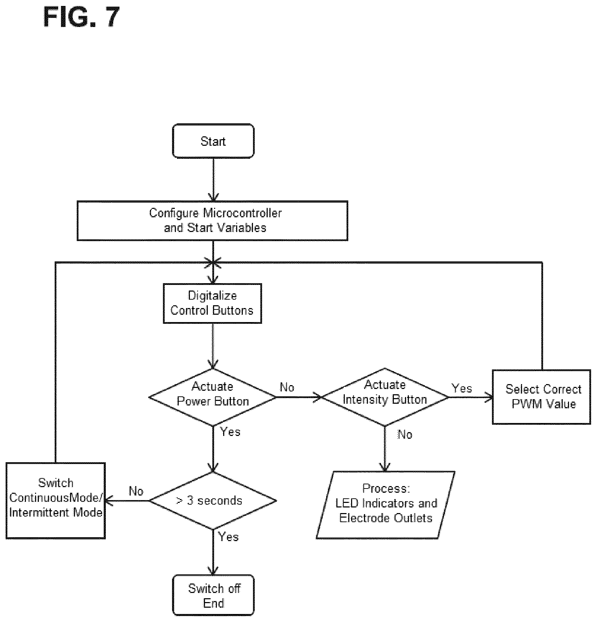

Figure 7 is a flowchart of the software, specifically developed for this innovative electrostimulation protocol.

As previously mentioned, by the novel protocol developed by the Inventor in addition to reducing significantly nerve fiber accommodation, it was possible to reduce the current consumption in operations carried out by the electrostimulation equipment, allowing the use of ordinary disposable coin-shaped lithium ion batteries, model CR20XX, which have smaller sizes, lower cost and lower capacity in mA/h, but which are sufficient to meet the current lower consumption required during random intensity variations, determined by the innovative electric stimulation protocol.

## Claims

1. A non-therapeutic PROCESS FOR ESTABLISHING AN ELECTROSTIMULATION PROTOCOL, **characterized in that** it provides a random intensity variation of electrical pulses, respecting the stimulation effectiveness limits, said random variation being comprised, at each chosen intensity level, within a variation range of 10 V with 500Ω charge, wherein said electrical pulses exhibit the shape of a square wave, are monopolar, and have a width or duration ranging between 60 µs and 100 µs, and frequency between 40 Hz and 70 Hz.

2. The PROCESS FOR ESTABLISHING AN ELECTROSTIMULATION PROTOCOL, according to claim 1, **characterized in that** said random intensity variation of the electrical pulses is comprised in the range of 5 V with 500 Ωcharge, that said width or duration of the electrical pulses is 80 µs, and/or that said frequency of the electrical pulses is 55 Hz.

3. The PROCESS FOR ESTABLISHING AN ELECTROSTIMULATION PROTOCOL, according to claim 1, **characterized in that** it provides two electrostimulation modes, namely, continuous mode and intermittent mode.

4. The PROCESS FOR ESTABLISHING AN ELECTROSTIMULATION PROTOCOL, according to claims 1 and 3, **characterized in that**, pulse bursts are continuously applied, in a continuous mode, with duration from 500 milliseconds to 2 seconds, and with sequentially inverted polarity.

5. The PROCESS FOR ESTABLISHING AN ELECTROSTIMULATION PROTOCOL, according to claim 4, **characterized in that** said pulse bursts exhibit a duration of 1 second.

6. The PROCESS FOR ESTABLISHING AN ELECTROSTIMULATION PROTOCOL, according to claims 1 and 3, **characterized in that** pulse bursts lasting 2-4 seconds and with sequentially inverted polarity, with a time intervals between the pulse bursts, are applied in an intermittent fashion, wherein said interval lasts 2-4 seconds.

7. The PROCESS FOR ESTABLISHING AN ELECTROSTIMULATION PROTOCOL, according to claim 6, **characterized in that** said pulse bursts exhibit a duration of 3 second.

8. The PROCESS FOR ESTABLISHING AN ELECTROSTIMULATION PROTOCOL, according to claim 6, **characterized in that** said time interval between the pulse bursts exhibits a duration of 3 second.

9. The PROCESS FOR ESTABLISHING AN ELECTROSTIMULATION PROTOCOL, according to claim 1, **characterized in that** it optionally discloses the simultaneous use of other strategies both to avoid the nerve fiber accommodation and to reduce current consumption, used together with the random variation of the pulse intensity.

10. The PROCESS FOR ESTABLISHING AN ELECTROSTIMULATION PROTOCOL, according to claims 1 and 9, **characterized in that** one of said strategy is the use of monopolar pulse bursts,
that the other of said strategies is pulse burst polarity inversion and/or that one of said strategy is the addition of intermittent stimulation modes.

11. The PROCESS FOR ESTABLISHING AN ELECTROSTIMULATION PROTOCOL, according to claims 1 and 10, **characterized in that** said strategies comprise the selection of different electrostimulation ranges, wherein all of them include a random variation range.

12. An ELECTROSTIMULATION PORTABLE EQUIPMENT adapted to apply SAID PROTOCOL according to any of the previous claims, of the type consisting of a bandage (1) comprising a central electronic module (2) and two side flaps (3), which house the respective electrodes, suitably covered by the respective gel layers, protected by the respective removable protective sheets, said central electronic module (2) housing the internal components and the electrical circuit of the equipment, as well as its power supply, wherein, in addition to an on-off button (4) and a led (5), signalizing that the equipment is working, one or more control buttons (6) can be externally provided, **characterized in that** said battery is a non-rechargeable disposable power battery and **in that** it houses the following internal components: Power source module (7), step-up regulator module (8), micro controller module (9), power supply seal module (10), boost source module (11), H-bridge module (12), electrode output module (13), and module for switching off when there is no charge (14).

13. The ELECTROSTIMULATION PORTABLE EQUIPMENT according to claim 12, **characterized in that** said battery is a 3V one.

## Patentansprüche

1. Nicht-therapeutischer Prozess zur Erstellung eines Elektrostimulationsprotokolls, **dadurch gekennzeichnet, dass** er eine zufällige Änderung der Intensität elektrischer Impulse bereitstellt, wobei er die Grenzen der Wirksamkeit einer Stimulation berücksichtigt, wobei die zufällige Änderung auf jedem gewählten Intensitätsniveau innerhalb eines Variationsbereichs von 10 V mit einer Ladung von 500 Ω enthalten ist, wobei die elektrischen Impulse die Form einer Rechteckwelle zeigen, monopolar sind und eine Breite oder Dauer im Bereich zwischen 60 µs und 100 µs und eine Frequenz zwischen 40 Hz und 70 Hz aufweisen.

2. Prozess zur Erstellung eines Elektrostimulationsprotokolls nach Anspruch 1, **dadurch gekennzeichnet, dass** die zufällige Intensitätsänderung der elektrischen Impulse im Bereich von 5 V mit einer Ladung von 500 Ω enthalten ist, dass die Breite oder die Dauer der elektrischen Impulse 80 µs beträgt und/oder dass die Frequenz der elektrischen Impulse 55 Hz beträgt.

3. Prozess zur Erstellung eines Elektrostimulationsprotokolls nach Anspruch 1, **dadurch gekennzeichnet, dass** er zwei Elektrostimulationsmodi bereitstellt, nämlich einen kontinuierlichen Modus und einen intermittierenden Modus.

4. Prozess zur Erstellung eines Elektrostimulationsprotokolls nach einem der Ansprüche 1 und 3, **dadurch gekennzeichnet, dass** in einem kontinuierlichen Modus Impuls-Bursts mit einer Dauer von 500 Millisekunden bis 2 Sekunden und mit einer sequenziell invertierten Polarität kontinuierlich angelegt werden.

5. Prozess zur Erstellung eines Elektrostimulationsprotokolls nach Anspruch 4, **dadurch gekennzeichnet, dass** die Impuls-Bursts eine Dauer von 1 Sekunde haben.

6. Prozess zur Erstellung eines Elektrostimulationsprotokolls nach einem der Ansprüche 1 und 3, **dadurch gekennzeichnet, dass** die Impuls-Bursts, die 2-4 Sekunden dauern und eine sequenziell invertierte Polarität aufweisen, mit Zeitintervallen zwischen den Impuls-Bursts, auf intermittierende Weise angelegt werden, wobei das Intervall 2-4 Sekunden lang ist.

7. Prozess zur Erstellung eines Elektrostimulationsprotokolls nach Anspruch 6, **dadurch gekennzeichnet, dass** die Impuls-Bursts eine Dauer von 3 Sekunde haben.

8. Prozess zur Erstellung eines Elektrostimulationsprotokolls nach Anspruch 6, **dadurch gekennzeichnet, dass** das Zeitintervall zwischen den Impuls-Bursts eine Dauer von 3 Sekunde hat.

9. Prozess zur Erstellung eines Elektrostimulationsprotokolls nach Anspruch 1, **dadurch gekennzeichnet, dass** er optional die gleichzeitige Verwendung anderer Strategien, sowohl zur Vermeidung einer Nevenfaserakkommodation als auch zur Verringerung eines Stromverbrauchs, die zusammen mit der zufälligen Änderung der Impulsintensität angewendet werden, offenbart.

10. Prozess zur Erstellung eines Elektrostimulationsprotokolls nach einem der Ansprüche 1 und 9, **dadurch gekennzeichnet, dass** eine der Strategien die Verwendung monopolarer Impuls-Bursts ist, dass die andere der Strategien eine Inversion von Impuls-Burst-Polaritäten ist und/oder dass die eine von den Strategien die Hinzufügung intermittierender Stimulationsmodi ist.

11. Prozess zur Erstellung eines Elektrostimulationsprotokolls nach einem der Ansprüche 1 und 10, **dadurch gekennzeichnet, dass** die Strategien die Auswahl unterschiedlicher Elektrostimulationsbereiche, die alle einen Zufallsänderungsbereich beinhalten, umfassen.

12. Tragbares Elektrostimulationsgerät, das dafür ausgelegt ist, das Protokoll nach einem der vorangehenden Ansprüche anzuwenden, von der Art, die aus einer Bandage (1) besteht, die ein mittleres elektronisches Modul (2) und zwei Seitenflügel (3) umfasst, in denen die jeweiligen Elektroden untergebracht sind, die auf geeignete Weise mit entsprechenden Gelschichten bedeckt sind, und die durch entsprechende abnehmbare schützende Flächengebilde bedeckt sind, wobei das zentrale elektronische Modul (2) die internen Komponenten und den Stromkreis des Geräts, ebenso wie dessen Stromquelle birgt, wobei zusätzlich zu einem Ein/Aus-Schalter (4) und einer LED (5), die signalisiert, dass das Gerät in Betrieb ist, ein oder mehrere Steuertasten (6) extern bereitgestellt sein können, **dadurch gekennzeichnet, dass** die Batterie eine nicht-wiederaufladbare Einwegbatterie ist und dass sie die folgenden internen Komponenten birgt: ein Stromquellenmodul (7), ein Hochreglermodul (8), ein Mikrocontroller-Modul (9), ein Stromquellendichtungsmodul (10), ein Boost-Source-Modul (11), ein H-Brücken-Modul (12), eine Elektrodenausgangsmodul (13) und ein Modul (14) zum Abschalten, wenn keine Ladung vorhanden ist.

13. Tragbares Elektrostimulationsgerät nach Anspruch 12, **dadurch gekennzeichnet, dass** die Batterie 3 V aufweist.

## Revendications

1. PROCEDE non thérapeutique DESTINE A L'ETABLISSEMENT D'UN PROTOCOLE D'ELECTROSTIMULATION, **caractérisé en ce qu'**il délivre une variation d'intensité aléatoire des impulsions électriques, respectant les limites d'efficacité de la stimulation, ladite variation aléatoire étant comprise, à chaque niveau d'intensité choisi, à l'intérieur d'une plage de variation de 10 V avec une charge de 500 Ω, lesdites impulsions électriques présentant une onde de forme carrée, sont unipolaires, et ont une largeur ou une durée qui s'étend entre 60 µs et 100 µs, et une fréquence entre 40 Hz et 70 Hz.

2. PROCEDE DESTINE A L'ETABLISSEMENT D'UN PROTOCOLE D'ELECTROSTIMULATION selon la revendication 1, **caractérisé en ce que** ladite variation d'intensité aléatoire des impulsions électriques est comprise dans le domaine de 5 V avec une charge de 500 Ω, **en ce que** ladite largeur ou durée des impulsions électriques est de 80 µs, et/ou **en ce que** ladite fréquence des impulsions électriques est de 55 Hz.

3. PROCEDE DESTINE A L'ETABLISSEMENT D'UN PROTOCOLE D'ELECTROSTIMULATION selon la revendication 1, **caractérisé en ce qu'**il délivre deux modes d'électrostimulation, en l'occurrence, un mode continu et un mode discontinu.

4. PROCEDE DESTINE A L'ETABLISSEMENT D'UN PROTOCOLE D'ELECTROSTIMULATION selon les revendications 1 et 3, **caractérisé en ce que** des paquets d'impulsions sont appliqués de manière continue, dans un mode continu, avec une durée allant de 500 millisecondes à 2 secondes, et avec une inversion séquentielle de la polarité.

5. PROCEDE DESTINE A L'ETABLISSEMENT D'UN PROTOCOLE D'ELECTROSTIMULATION selon la revendication 4, **caractérisé en ce que** lesdits paquets d'impulsions présentent une durée d'une seconde.

6. PROCEDE DESTINE A L'ETABLISSEMENT D'UN PROTOCOLE D'ELECTROSTIMULATION selon les revendications 1 et 3, **caractérisé en ce que** des paquets d'impulsions durant de 2 à 4 secondes et avec une inversion séquentielle de la polarité, avec un intervalle de temps entre les paquets d'impulsions, sont appliqués de manière discontinue, où ledit intervalle dure de 2 à 4 secondes.

7. PROCEDE DESTINE A L'ETABLISSEMENT D'UN PROTOCOLE D'ELECTROSTIMULATION selon la revendication 6, **caractérisé en ce que** lesdits paquets d'impulsions présentent une durée de 3 secondes.

8. PROCEDE DESTINE A L'ETABLISSEMENT D'UN PROTOCOLE D'ELECTROSTIMULATION selon la revendication 6, **caractérisé en ce que** ledit intervalle de temps entre les paquets d'impulsions présentent une durée de 3 secondes.

9. PROCEDE DESTINE A L'ETABLISSEMENT D'UN PROTOCOLE D'ELECTROSTIMULATION selon la revendication 1, **caractérisé en ce qu'**il révèle éventuellement l'utilisation simultanée d'autres stratégies pour a la fois éviter l'accommodation de la fibre nerveuse et diminuer la consommation de courant, utilisées en association avec la variation aléatoire de l'intensité des impulsions.

10. PROCEDE DESTINE A L'ETABLISSEMENT D'UN PROTOCOLE D'ELECTROSTIMULATION selon les revendications 1 et 9, **caractérisé en ce que** l'une desdites stratégies est l'utilisation de paquets d'impulsions unipolaires, **en ce que** l'autre parmi lesdites stratégies est l'inversion de la polarité des paquets d'impulsions et/ou **en ce que** l'une desdites stratégies est l'ajout de modes de stimulation discontinus.

11. PROCEDE DESTINE A L'ETABLISSEMENT D'UN PROTOCOLE D'ELECTROSTIMULATION selon les revendications 1 et 10, **caractérisé en ce que** lesdites stratégies comprennent la sélection de différentes plages d'électrostimulation, où chacune d'entre elles comprend une plage de variation aléatoire.

12. EQUIPEMENT PORTATIF D'ELECTROSTIMULATION permettant d'appliquer LEDIT PROTOCOLE selon l'une quelconque des revendications précédentes, du type consistant en un bandage (1) comprenant un module électronique central (2), et deux rabats latéraux (3), qui abritent les électrodes respectives, recouvertes de manière appropriée par les couches de gel respectives, protégées par des feuilles de protection amovibles respectives, ledit module électronique central (2) abritant les composants internes et le circuit électrique de l'équipement, ainsi que son alimentation électrique, où, en plus d'une touche marche - arrêt (4) et d'une diode électroluminescente (5), indiquant que l'équipement est en fonctionnement, une ou plusieurs touches de commande (6) peuvent être placées extérieurement, **caractérisé en ce que** ladite pile est une pile jetable non rechargeable et **en ce qu'**il abrite les composants internes suivants : un module de la source d'alimentation (7), un module régulateur du système (8), un module du microsystème de commande (9), un module d'étanchéité de l'alimentation électrique (10), un module de la source de stimulation (11), un module de pont en H (12), un module de sortie d'électrode (13) et un module de mise hors tension quand il n'y a pas de charge (14).

13. EQUIPEMENT PORTATIF D'ELECTROSTIMULATION selon la revendication 12, **caractérisé en ce que** ladite pile est une pile de 3 V.
